(19) Europäisches Patentamt
European Patent Office
Office européen des brevets

(11) Veröffentlichungsnummer: **0 213 474 B1**

# (12) EUROPÄISCHE PATENTSCHRIFT

(45) Veröffentlichungstag der Patentschrift: **03.02.93**

(21) Anmeldenummer: **86111104.5**

(22) Anmeldetag: **12.08.86**

Die Akte enthält technische Angaben, die nach dem Eingang der Anmeldung eingereicht wurden und die nicht in dieser Patentschrift enthalten sind.

(51) Int. Cl.⁵: **C07D 235/28**, C07D 403/12, C07D 401/12, C07D 491/04, A61K 31/415, A61K 31/47

## (54) Substituierte Toluidine, Verfahren zu ihrer Herstellung, diese enthaltende pharmazeutische Zubereitungen und ihre Verwendung als Magensäuresekretionshemmer.

(30) Priorität: **24.08.85 DE 3530342**
**29.03.86 DE 3610609**

(43) Veröffentlichungstag der Anmeldung:
**11.03.87 Patentblatt 87/11**

(45) Bekanntmachung des Hinweises auf die Patenterteilung:
**03.02.93 Patentblatt 93/05**

(84) Benannte Vertragsstaaten:
**AT BE CH DE FR GB IT LI LU NL SE**

(56) Entgegenhaltungen:
**EP-A- 045 200**
**EP-A- 074 341**
**EP-A- 0 174 717**
**EP-A- 0 204 215**
**WO-A-87/01114**
**DE-A- 3 531 487**

**Nature, vol. 290 (1981) Seite 159**

**TIPS, vol. 5 (1984) Seite 262**

**Scand. J. Gastroent., vol. 20, Suppl. 108 (1985), Seite 15-22**

(73) Patentinhaber: **HOECHST AKTIENGESELLSCHAFT**
**Postfach 80 03 20**
**W-6230 Frankfurt am Main 80(DE)**

(72) Erfinder: **Lang, Hans-Jochen, Dr.**
**Rüdesheimer Strasse 7**
**W-6238 Hofheim am Taunus(DE)**
Erfinder: **Rackur, Gerhard, Dr.**
**Weimarer Strasse 17**
**W-6270 Idstein(DE)**
Erfinder: **Rösner, Manfred, Dr.**
**Unter den Buchen 7**
**W-6239 Eppstein/Taunus(DE)**
Erfinder: **Herling, Andreas W.**
**Dieburger Strasse 43**
**W-6072 Dreieich(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 213 474 B1

## Beschreibung

Benzimidazolderivate mit schmerzstillender Wirkung sind aus der DE-A-22 46 429, solche mit magensäuresekretionshemmender Wirkung sind z. B. aus DE-A-25 48 340, EP-A-5129, DE-A-32 40 248 und DE-A-33 33 314 bekannt. Weitere Benzimidazolderivate sind in der deutschen Patentanmeldung P 35 09 333.1 vorgeschlagen worden.

DE-A-35 31 487 (offengelegt: 13.3.1986), EP-A-174 717 (offengelegt: 19.3.1986) und EP-A-0 204 215 (offengelegt: 10.12.1986) betreffen ebenfalls Benzimidazolderivate.

In EP-A-0 045 200 und EP-A-0 074 341 werden zur Behandlung von entzündlichen gastrointestinalen Erkrankungen bzw. zur Inhibition der Magensäuresekretion Benzimidazolderivate vorgeschlagen, die aus 3 wichtigen Strukturelementen bestehen: einem substituierten Benzimidazolring, einem substituierten Heterocyclus oder Benzolkern und einer schwefelhaltigen Kette zwischen den beiden Aromaten. Tierexperimentelle Wirksamkeitsstudien mit ähnlichen Verbindungen, wie Omeprazol, Picoprazol und deren Derivaten wurden im Scand. J.Gastroenterol 1985; 20(suppl 108): 15-22 publiziert. Sie führten zu der Erkenntnis, daß alle drei obengenannten Strukturelemente für die pharmakologische Wirksamkeit der Benzimidazolderivate von gleich wesentlicher Bedeutung sind und daß, bezogen auf Omeprazol als Referenz, Modifikationen am Pyridinring oder Versuche, eines der o.g. Strukturelemente durch ein anderes zu ersetzen, in allen Fällen zu einer meist entscheidend geringeren biologischen Wirksamkeit führten.

Der vorliegenden Erfindung liegt daher die Aufgabe zugrunde, eine weitere Substanz bereitzustellen mit, gemessen an bekannten Stoffen, mindestens gleicher oder besserer biologischer Wirksamkeit in dem oben genannten Sinne. Es wurde nun überraschenderweise gefunden, daß bestimmte substituierte Toluidine hochwirksame Magensäuresekretionshemmer sind.

Die vorliegende Erfindung betrifft o-Toluidinderivate der Formel I

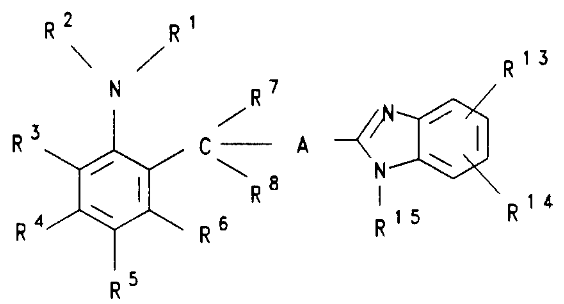

(I)

in welcher

| | |
|---|---|
| A | für -S- oder -SO- steht, |
| $R^1$ | $(C_1-C_4)$-Alkyl, vorzugsweise Methyl oder Ethyl bedeutet, |
| $R^2$,$R^7$, $R^8$ und $R^{15}$ | jeweils Wasserstoff bedeuten, |
| $R^3$,$R^4$,$R^5$ und $R^6$ | gleich oder verschieden sind und Wasserstoff, $(C_1-C_4)$-Alkyl oder |

Methoxy bedeuten oder $R^4$ und $R^5$ zusammen für -CH=CH-CH=CH- stehen, $R^{13}$ und $R^{14}$ gleich oder verschieden sind und $(C_1-C_4)$-Alkoxy bedeuten, sowie deren physiologisch verträglichen Salze.

Bevorzugt sind Verbindungen der Formel I, in welcher A für -SO- steht, sowie deren physiologisch verträglichen Salze.

Bevorzugt sind insbesondere Verbindungen der Formel I, in welcher $R^3$, $R^4$, $R^5$ und $R^6$ jeweils für Wasserstoff stehen, sowie deren physiologisch verträglichen Salze.

Weiterhin bevorzugt sind Verbindungen der Formel I, in welcher $R^{13}$ und $R^{14}$ in der 5- bzw. 6-Position des Benzimidazol-Systems stehen, gleich oder verschieden sind und Methoxy oder Ethoxy, vorzugsweise Methoxy bedeuten und $R^1$ Ethyl bedeutet, sowie deren verträglichen Salze.

Hervorgehoben seien weiterhin Verbindungen der Formel I, worin ein oder zwei Reste $R^3$, $R^4$, $R^5$ und $R^6$ für $(C_1-C_4)$-Alkyl steht (stehen), sowie deren physiologisch verträglichen Salze.

Ganz besonders bevorzugt ist 2-(2-Ethylamino-benzylsulfinyl)-5,6-dimethoxybenzimidazol und dessen physiologisch verträglichen Salze.

Gegebenenfalls vorhandene chirale C- oder S-Atome können sowohl in der R- als auch in der S-Konfiguration vorkommen. In solchen Fällen liegen Verbindungen der Formel I in Form der reinen Enantiomeren oder als Stereoisomerengemisch (wie Enantiomerengemisch und Diastereomerengemisch) vor.

Als Salze kommen insbesondere Alkali- und Erdalkalisalze und Salze anorganischer oder organischer Säuren, wie z.B. HCl, HBr, $H_2SO_4$, Methansulfonsäure, Amidosulfonsäure, p-Toluolsulfonsäure in Frage.

Alkyl und Alkoxy können geradkettig oder verzweigt sein.

Die Erfindung betrifft weiterhin ein Verfahren zur Herstellung einer Verbindung der Formel I, dadurch gekennzeichnet, daß man

a) eine Verbindung der Formel II

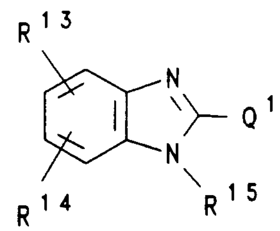

( I I )

in welcher $R^{13}$, $R^{14}$ und $R^{15}$ wie oben definiert sind und

$Q^1$ i. eine Abgangsgruppe oder
ii. -SH, -S- oder $SO_2$- bedeutet,

umsetzt mit einer Verbindung der Formel III

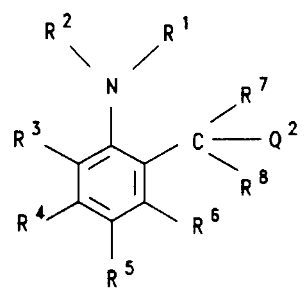

( I I I )

in welcher $R^1$, $R^2$, $R^3$, $R^4$, $R^5$, $R^6$, $R^7$ und $R^8$ wie oben definiert sind, und

$Q^2$ im obengenannten Fall i. -SH, -S- oder -$SO_2$-, im obengenannten Fall ii. eine Abgangsgruppe bedeutet,

b) eine Verbindung der Formel IV

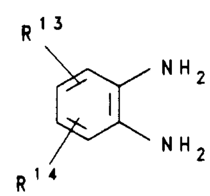

( I V )

in welcher $R^{13}$ und $R^{14}$ wie oben definiert sind, umsetzt mit einer Verbindung der Formel V

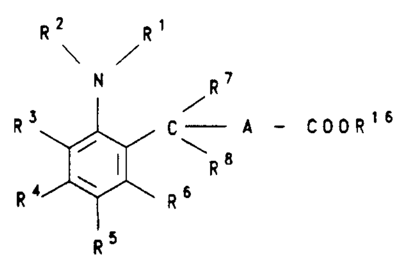

( V )

in welcher $R^1$, $R^2$, $R^3$, $R^4$, $R^5$, $R^6$, $R^7$, $R^8$ und A wie oben definiert sind, und $R^{16}$ für eine veresternde Gruppe steht, oder

c) zur Herstellung einer Verbindung der Formel I, worin $R^1$ und $R^2$ jeweils Wasserstoff bedeutet, eine Verbindung der Formel VI

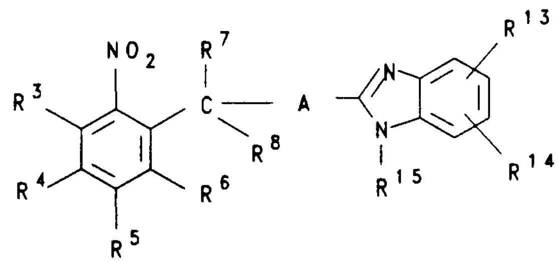

(VI)

in welcher $R^3$, $R^4$, $R^5$, $R^6$, $R^7$, $R^8$, $R^{13}$, $R^{14}$ und $R^{15}$ wie oben definiert sind und A für ein Schwefelatom steht, reduziert,

in den nach a), b) oder c) erhaltenen Verbindungen der Formel I, in welcher A für -S- oder-SO- und/oder $R^3$ bis $R^6$ und/oder $R^{13}$, $R^{14}$ für -S- oder-SO-haltige Reste stehen, zur entsprechenden -SO- oder-$SO_2$-Gruppe oxidiert,

eine Verbindung der Formel I worin $R^1$ und/oder $R^2$ für Wasserstoff steht, alkyliert oder acyliert,

eine Verbindung der Formel I, worin $R^1$ und/oder $R^2$ für Acyl steht, einer desacylierenden Hydrolyse oder Hydrogenolyse unterwirft,

eine so erhaltene Verbindung der Formel I gegebenenfalls in ihr physiologisch verträgliches Salz überführt.

Setzt man gemäß der hier bevorzugten Verfahrensvariante a) Verbindungen der Formel II mit Verbindungen der Formel III um, so steht $Q^1$ oder $Q^2$ für eine Abgangsgruppe, die nucleophil ablösbar ist, wie Cl, Br, J, $-OSO_2CH_3$, $-O-SO_2CF_3$ oder $-O-SO_2C_6H_4-pCH_3$.

Die Umsetzung einer Verbindungen der Formel II mit einer Verbindung der Formel III oder deren Salzen erfolgt in einem inerten Lösungsmittel wie z.B. Wasser, Methanol, Ethanol, Isopropanol, Tetrahydrofuran, Dioxan, 1,2-Dimethoxyethan, Methylenchlorid, Aceton, Essigsäureethylester, Dimethylformamid, Acetonitril, Dimethylacetamid, Dimethylsulfoxid, oder Gemischen dieser Lösungsmittel. Die Reaktion kann in An- und Abwesenheit einer anorganischen oder organischen Base durchgeführt werden, wobei Natrium- oder Kaliumhydroxid, -carbonat, -alkoxid, -hydrid, -amid, Ammoniak, Triethylamin, Tributylamin, Pyridin verwendet werden können. Die Reaktionstemperatur liegt zwischen -20 und + 150 °C, vorzugsweise bei 0 bis 80 °C.

Verbindungen der Formel II sind literaturbekannt oder können in Analogie zu bekannten Verfahren hergestellt werden, z.B. durch Ringschluß entsprechend substituierter o-Phenylendiamine mit Schwefelkohlenstoff (z.B. DE-A-31 32 167).

Die hierfür benötigten o-Phenylendiamine sind ebenfalls literaturbekannt und werden z.B. durch katalytische Reduktion entsprechend substituierter o-Nitroaniline erhalten.

Verbindungen der Formel III sind ebenfalls literaturbekannt oder können in Analogie zu bekannten Verfahren hergestellt werden.

In den bei Verfahrensvariante b) eingesetzten Estern der Formel V steht $R^{16}$ für eine veresternde Gruppe vorzugsweise ($C_1$-$C_6$)-Alkyl oder Benzyl. Die Umesterung der Verbindungen der Formel IV mit Verbindungen der Formel V erfolgt analog der in Preston et.al., Benzimidazoles and Congeneric Tricyclic Compounds, Part 1, New York, Seiten 10-13 beschriebenen Verfahrensweisen.

Als besonders geeignete Reduktionsmittel für Verbindungen der Formel VI erwiesen sich Zinn(II)salze wie $SnCl_2$. Besonders vorteilhaft wird die Reduktion im wäßrigen Medium oder in Mischungen einer wäßrigen Phase mit polaren organischen Lösungsmittel unter stark sauren Bedingungen, beispielsweise in konzentrierter Salzsäure zwischen O und 100 °C, vorteilhaft zwischen 20 und 80 °C, durchgeführt. Aufarbeitung und Isolierung der Substanz geschieht vorteilhaft unter alkalischen Bedingungen in an sich literaturbekannter Weise.

Die als Ausgangsprodukte verwendeten 2-(2-Nitrobenzylthio)-benzimidazolderivateerhält man entsprechend der Verfahrensweise a) ii. in an sich bekannter Weise durch Umsetzung eines 2-Mercaptobenzimidazols der Formel II mit einem 2-Nitrobenzylhalogenid, vorzugsweise mit einem 2-Nitrobenzylchlorid- oder -bromidderivat der Formel VII (Hal = Cl oder Br).

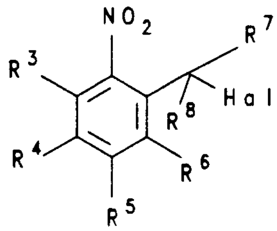

(VII)

Die so erhaltenen Verbindungen der Formel I, worin A für -S- steht können, falls $R^3$ Wasserstoff bedeutet, mit Basen in physiologisch verträgliche Salze umgewandelt werden.

Verbindungen der Formel I mit A= -S- können ferner mit geeigneten Oxidationsmitteln in solche mit A= -SO- oder $-SO_2-$ umgewandelt werden.

Diese Reaktion erfolgt in einem geeigneten, inerten Lösungsmittel wie z.B. Methylenchlorid, Chloroform, Tetrachlor-Kohlenstoff, 1,2-Dichlorethan, Toluol, Essigsäureethylester, Essigsäure, Trifluoressigsäure, Wasser, Methanol, Ethanol oder Gemischen derselben bei -20°C bis +150°C vorzugsweise bei -10°C bis +40°C.

Als Oxidationsmittel kommen z.B. in Betracht:
Wasserstoffperoxid, Persäuren und Perester, wie Peressigsäure, Trifluorperessigsäure, Monoperphthalsäure, m-Chlorperbenzoesäure und deren Ester, Ozon, Distickstofftetroxid, Jodosobenzol, N-Chlorsuccinimid, 1-Chlorbenzotriazol, Natriumhypochlorit, Kaliumperoxodisulfat, t-Butylhypochlorit, Tetrabutylammoniumperjodat oder -permanganat, Natrium-meta-perjodat, Selen- oder Mangandioxid, Cerammonnitrat, Chromsäure, Chlor, Brom, Diazabicyclo[2,2,2]octan-Bromkomplex, Dioxandibromid, Pyridiniumperbromid.

Ebenso können isolierte, gegebenenfalls immobilisierte oxidierende Enzyme oder Mikroorganismen als Oxidationsmittel Anwendung finden.

Die Oxidationsmittel werden in äquimolaren Mengen, ggf. auch in einem geringen Überschuß von 5-10 Mol-% bei der Oxydation zu A= -SO- oder auch in größerem Überschuß und/oder bei höherer Reaktionstemperatur eingesetzt, wenn eine Oxydation zu A= $-SO_2-$ gewünscht wird.

Als erfindungsgemäße Verbindungen seien genannt:

$R^7$, $R^8$, $R^{15}$ = H

A = S

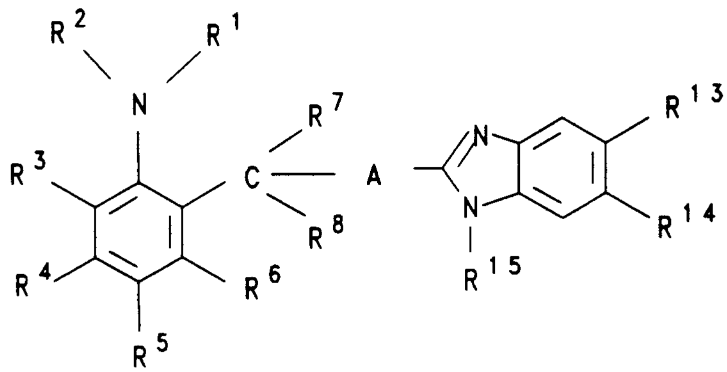

| $R^1$ | $R^2$ | $R^3$ | $R^4$ | $R^5$ | $R^6$ | $R^{13}$ | $R^{14}$ |
|---|---|---|---|---|---|---|---|
| Me | H | H | Me | H | H | OMe | OMe |
| Me | H | H | H | Me | H | OMe | OMe |
| Me | H | H | H | OMe | Me | OMe | OMe |
| Me | H | Me | H | OMe | H | OMe | OMe |
| Me | H | Me | H | H | H | OMe | OMe |
| Me | H | H | Me | H | H | OMe | OMe |
| Me | H | H | H | Me | H | OMe | OMe |
| Me | H | Et | H | H | H | OMe | OMe |
| Me | H | H | Et | H | H | OMe | OMe |
| Me | H | H | H | Et | H | OMe | OMe |

| $R^1$ | $R^2$ | $R^3$ | $R^4$ | $R^5$ | $R^6$ | $R^{13}$ | $R^{14}$ |
|---|---|---|---|---|---|---|---|
| Me | H | H | H | H | Me | OMe | OMe |
| Et | H | H | H | OMe | Me | OMe | OMe |
| Et | H | Me | H | OMe | H | OMe | OMe |
| Et | H | Me | H | H | H | OMe | OMe |
| Et | H | H | Me | H | H | OMe | OMe |
| Et | H | H | H | Me | H | OMe | OMe |
| Et | H | Et | H | H | H | OMe | OMe |
| Et | H | H | Et | H | H | OMe | OMe |
| Et | H | H | H | Et | H | OMe | OMe |
| Et | H | H | H | H | Me | OMe | OMe |
| Et | H | OMe | H | H | H | OMe | OMe |
| Et | H | H | H | OMe | H | OMe | OMe |
| Et | H | Me | H | Me | H | OMe | OMe |
| Et | H | Me | Me | H | H | OMe | OMe |
| Et | H | H | Me | Me | H | OMe | OMe |
| Me | H | H | OMe | H | H | OMe | OMe |
| Me | H | H | H | OMe | H | OMe | OMe |
| Me | H | Me | H | Me | H | OMe | OMe |
| Me | H | Me | Me | H | H | OMe | OMe |
| Me | H | H | Me | Me | H | OMe | OMe |
| Me | H | H | Me | Me | Me | OMe | OMe |
| Me | H | H | OMe | Me | Me | OMe | OMe |
| Me | H | H | Me | OMe | Me | OMe | OMe |

$R^7$, $R^8$, $R^{15}$ = H

A = SO

| $R^1$ | $R^2$ | $R^3$ | $R^4$ | $R^5$ | $R^6$ | $R^{13}$ | $R^{14}$ |
|---|---|---|---|---|---|---|---|
| Et | H | Me | H | Me | H | OMe | OMe |
| Et | H | Me | Me | H | H | OMe | OMe |
| Et | H | H | Me | Me | H | OMe | OMe |

| $R^1$ | $R^2$ | $R^3$ | $R^4$ | $R^5$ | $R^6$ | $R^{13}$ | $R^{14}$ |
|---|---|---|---|---|---|---|---|
| Me | H | H | Me | H | H | OMe | OMe |
| Me | H | H | H | Me | H | OMe | OMe |
| Me | H | Me | H | OMe | H | OMe | OMe |
| Me | H | Me | H | H | H | OMe | OMe |
| Me | H | H | Me | H | H | OMe | OMe |
| Me | H | H | H | Me | H | OMe | OMe |
| Me | H | Et | H | H | H | OMe | OMe |
| Me | H | H | Et | H | H | OMe | OMe |
| Me | H | H | H | Et | H | OMe | OMe |
| Me | H | H | H | H | Me | OMe | OMe |
| Me | H | OMe | H | H | H | OMe | OMe |
| Et | H | H | H | OMe | Me | OMe | OMe |
| Et | H | Me | H | OMe | H | OMe | OMe |
| Et | H | Me | H | H | H | OMe | OMe |
| Et | H | H | Me | H | H | OMe | OMe |
| Et | H | H | H | Me | H | OMe | OMe |
| Et | H | Et | H | H | H | OMe | OMe |
| Et | H | H | Et | H | H | OMe | OMe |
| Et | H | H | H | Et | H | OMe | OMe |
| Et | H | H | H | H | Me | OMe | OMe |
| Et | H | OMe | H | H | H | OMe | OMe |
| Et | H | H | OMe | H | H | OMe | OMe |
| Et | H | H | H | OMe | H | OMe | OMe |
| Me | H | H | OMe | H | H | OMe | OMe |
| Me | H | H | H | OMe | H | OMe | OMe |
| Me | H | Me | H | Me | H | OMe | OMe |
| Me | H | Me | Me | H | H | OMe | OMe |
| Me | H | H | Me | Me | H | OMe | OMe |
| Me | H | H | Me | Me | Me | OMe | OMe |
| Me | H | H | OMe | Me | Me | OMe | OMe |
| Me | H | H | Me | OMe | Me | OMe | OMe |

## Abkürzungen:

**Me** **Methyl**

**Et** **Ethyl**

Die neuen Verbindungen der Formel I und ihre Salze besitzen wertvolle pharmakologische Eigenschaften.

Sie hemmen deutlich die Magensäurekretion und weisen darüber hinaus eine ausgezeichnete Magen- und Darmschutzwirkung auf.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. Antiphlogistika und Antirheumatika), Chemikalien (z.B. Ethanol) Magensäure oder Streßsituationen verursacht werden können.

Aufgrund Ihrer ausgezeichneten Eigenschaften sind die Verbindungen der Formel I und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Die Erfindung betrifft daher weiter die erfindungsgemäßen Verbindungen der Formel I zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die eine oder mehrere Verbindung(en) der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt vorteilhafterweise zwischen 0,1 und 96% beträgt.

Welche Hilfsstoffe für die gewünschte Arzneimittelformulierung geeignet sind, ist dem Fachmann aufgrund seines Wissens geläufig. Neben Lösemitteln, Gelbbildnern, Suppositoriengrundlagen, Tabletten-Hilfsstoffen und anderen Wirstoffträgrern können beispielweise Antioxidatien, Dispergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale Applikation bevorzugt ist.

Im allgemeinen hat es sich in der Humanmedizin als vorteilhaft erwiesen, den oder die Wirkstoff(e) bei oraler Gabe in einer Tagesdosis von etwa 0,01 bis etwa 20 mg/kg Körpergewicht, gegebenenfalls in Form mehrerer, vorzugsweise 1 bis 4 Einzelgaben zur Erzielung des gewünschten Ergebnisses zu verabreichen. Bei der parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der obengenannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Tranquilizer, wie Benzodiazepine, beispielsweise Diazepam; Spasmolytika, wie z.B. Bietamiverin, Camylofin; Anticholinergica, wie z.B. Oxyphencylimin, Phencarbamid; Lokalanaesthetike, wie z.B. Tetracain, Procain; gegebenenfalls auch Fermente, Vitamine oder Aminosäuren enthalten.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Dareichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel

kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propanol oder Glyzerin, daneben auch Zuckerlösungen wie Glucose- oder Mannitlösungen, oder auch eine Mischung aus den verschiedenen genannten Lösungsmitteln.

Die nachfolgenden Beispiele sollen die erfindungsgemäßen Verfahrensweisen erläutern.

Beispiel 1:

2-(2-Ethylaminobenzylthio)-5,6-dimethoxy-benzimidazol

10,5g 5,6-Dimethoxy-2-mercaptobenzimidazol werden in 250 ml THF suspendiert und portionsweise mit 15g 2-Ethylamino-benzylbromid-hydrobromid versetzt. Man rührt sechs Stunden bei Raumtemperatur, saugt dann den Niederschlag ab, wäscht ihn mit THF und Petrolether und trocknet unter vermindertem Druck bei ca. 80 ° C.

Das entstandene Hydrobromid wird in 400 ml 1%ige wäßrige Natriumhydroxidlösung eingetragen und zwei Stunden bei Raumtemperatur nachgerührt. Die freie Base wird abgesaugt, mit Wasser gewaschen und getrocknet.
Fp.162 ° C (Zers.).

Beispiel 2:

2-(2-Ethylaminobenzylsulfinyl)-5,6-dimethoxybenzimidazol

Zu 3,4g 2-(2-Ethylaminobenzylthio)-5,6-dimethoxybenzimidazol in 100 ml Methylenchlorid werden unter Rühren innerhalb von 10 Minuten 2,0 g 85%ige m-Chlorperbenzoesäure, gelöst in 70 ml Methylenchlorid, bei Raumtemperatur zugetropft. Man rührt 20 Minuten nach, versetzt mit überschüssiger zweinormaler wäßriger Ammoniak-Lösung und trennt die organische Phase ab. Nach Trocknen über Natriumsulfat wird einrotiert, mit wenig Aceton verrührt, abgesaugt, aus Aceton umkristallisiert und getrocknet.
Fp. 168 ° C (Zers.).

Beispiel 3:

Allgemeine Vorschrift zur Herstellung der freien Benzimidazolderivate aus den Benzimidazol-Natriumsalzen

Zur Überführung der Natriumsalze in die freien Sulfinylbenzimidazole der allgemeinen Formel I eignet sich ein Gemisch aus gleichen Teilen Tetrahydrofuran und gesättigter $NaHCO_3$-Lösung. Die organische Phase wird eingeengt und das kristalline Produkt mit Diisopropylether gewaschen.

In analoger Verfahrensweise werden die folgenden Verbindungen der Formel I hergestellt:

Tabelle

| Bsp. | $R^1$ | $R^2$ | $R^3$ | $R^4$ | $R^5$ | $R^6$ | $R^7$ | $R^8$ | A | | Fp. (°C) |
|---|---|---|---|---|---|---|---|---|---|---|---|
| | | | | | | | | | | 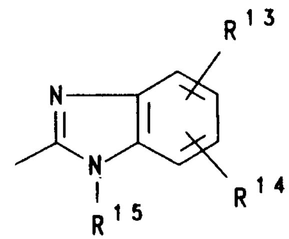 | |
| 4 | $C_2H_5$ | H | $CH_3$ | H | H | H | H | H | S | 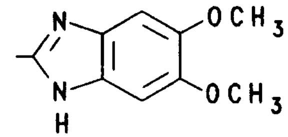 | 88 |
| 5 | $C_2H_5$ | H | $CH_3$ | H | H | H | H | H | SO | 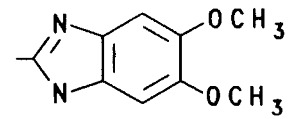 | 151 (Zers.) |
| 6 | $C_2H_5$ | H | H | H | H | $CH_3$ | H | H | S | 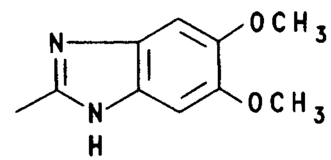 | 276 |
| 7 | $C_2H_5$ | H | H | H | H | $CH_3$ | H | H | SO | 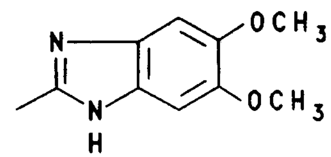 | 136 (Zers.) |

Fortsetzung Tabelle

| Bsp. | $R^1$ | $R^2$ | $R^3$ | $R^4$ | $R^5$ | $R^6$ | $R^7$ | $R^8$ | A | 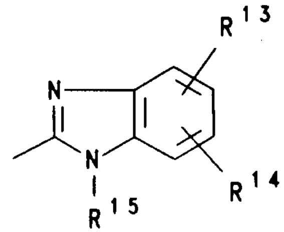 | Fp. (°C) |
|---|---|---|---|---|---|---|---|---|---|---|---|
| 8 | $CH_3$ | H | H | H | H | H | H | H | S | 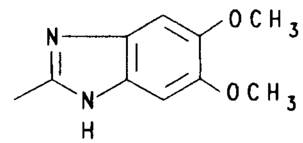 x 2 HBr | 254 (Zers.) |
| 9 | $CH_3$ | H | H | H | H | H | H | H | S | 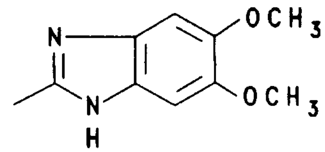 | 230 (Zers.) |
| 10 | $CH_3$ | H | H | H | H | H | H | H | SO | 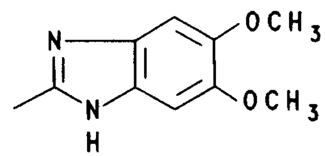 | 140 (Zers.) |
| 11 | $C_2H_5$ | H | H | H | H | H | H | H | S | 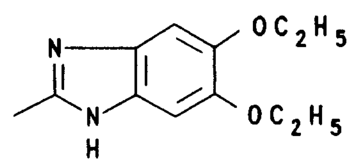 | 130 (Zers.) |

Fortsetzung Tabelle

| Bsp. | $R^1$ | $R^2$ | $R^3$ | $R^4$ | $R^5$ | $R^6$ | $R^7$ | $R^8$ | A | 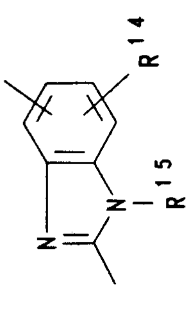 | Fp. (°C) |
|---|---|---|---|---|---|---|---|---|---|---|---|
| 12 | $C_2H_5$ | H | H | H | H | H | H | H | SO | 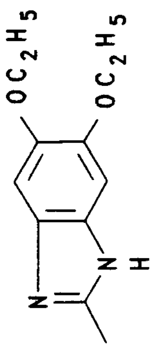 | ca. 156 (Zers.) |

**Patentansprüche**

**Patentansprüche für folgende Vertragsstaaten : BE, CH, DE, FR, GB, IT, LI, LU, NL, SE**

**1.** Verbindung der Formel I

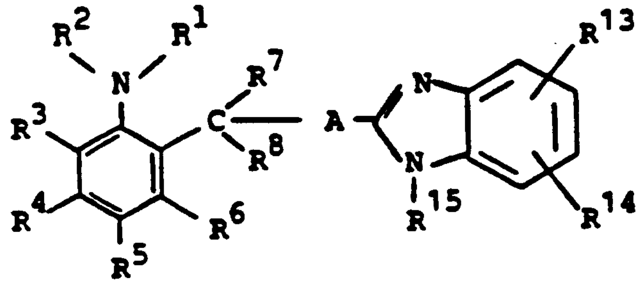

(I)

in welcher

| | |
|---|---|
| A | für -S- oder -SO- steht, |
| $R^1$ | $(C_1-C_4)$-Alkyl, vorzugsweise Methyl oder Ethyl bedeutet, |
| $R^2$, $R^7$, $R^8$ und $R^{15}$ | jeweils Wasserstoff bedeuten, |
| $R^3$,$R^4$,$R^5$ und $R^6$ | gleich oder verschieden sind und Wasserstoff, $(C_1-C_4)$-Alkyl oder Methoxy bedeuten oder $R^4$ und $R^5$ zusammen für -CH=CH-CH=CH- stehen, |
| $R^{13}$ und $R^{14}$ | gleich oder verschieden sind und $(C_1-C_4)$-Alkoxy bedeuten, sowie deren physiologisch verträglichen Salze. |

**2.** Verbindung der Formel I gemäß Anspruch 1 , in welcher A für -SO- steht, sowie deren physiologisch verträglichen Salze.

**3.** Verbindung der Formel I gemäß Anspruch 1 oder 2, in welcher $R^3$, $R^4$, $R^5$ und $R^6$ jeweils für Wasserstoff stehen, sowie deren physiologisch verträglichen Salze.

**4.** Verbindung der Formel I gemäß einem der Ansprüche 1 bis 3, in welcher $R^{13}$ und $R^{14}$ in der 5- bzw. 6-Position des Benzimidazol-Systems stehen, gleich oder verschieden sind und Methoxy oder Ethoxy, vorzugsweise Methoxy bedeuten und $R^1$ Ethyl bedeutet,
sowie deren physiologisch verträglichen Salze.

**5.** 2-(2-Ethylamino-benzylsulfinyl)-5,6-dimethoxybenzimidazol und dessen physiologisch verträglichen Salze.

**6.** Verbindung der Formel I gemäß einem der Ansprüche 1, bis 4 , in welcher ein oder zwei der Reste $R^3$, $R^4$, $R^5$ und $R^6$ für $(C_1-C_4)$-Alkyl steht (stehen), sowie deren physiologisch verträglichen Salze.

**7.** Verfahren zur Herstellung einer Verbindung der Formel I gemäß einem der Ansprüche 1 bis 6 dadurch gekennzeichnet, daß man
a) eine Verbindung der Formel II

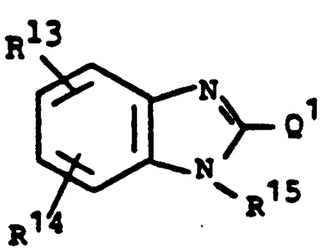

(II)

in welcher $R^{13}$, $R^{14}$ und $R^{15}$ wie in Anspruch 1 definiert sind und
$Q^1$ i. eine Abgangsgruppe oder
ii. -SH, $-S^-$ oder $-SO_2^-$ bedeutet,
umsetzt mit einer Verbindung der Formel III

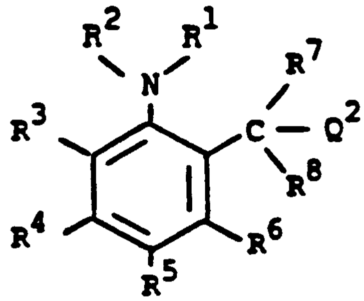

(III)

in welcher $R^1$, $R^2$, $R^3$, $R^4$, $R^5$, $R^6$, $R^7$ und $R^8$ wie im Anspruch 1 definiert sind, und

$Q^2$ im obengenannten Fall i. -SH, $-S^-$ oder $-SO_2^-$,
im obengenannten Fall ii. eine Angangsgruppe bedeutet,

b) eine Verbindung der Formel IV

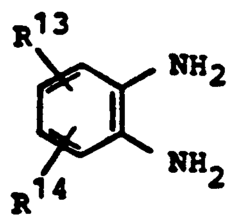

(IV)

in welcher $R^{13}$ und $R^{14}$ wie oben definiert sind, umsetzt, mit einer Verbindung der Formel V

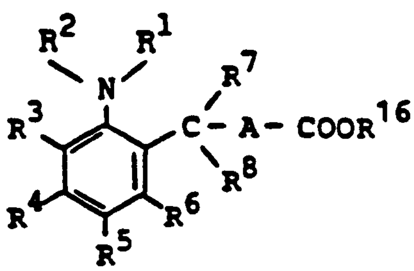

(V)

in welcher $R^1$, $R^2$, $R^3$, $R^4$, $R^5$, $R^6$, $R^7$, $R^8$ und A wie oben definiert sind und
$R^{16}$ für eine veresternde Gruppe steht, oder

c) zur Herstellung einer Verbindung der Formel I, worin $R^1$ und $R^2$ jeweils Wasserstoff bedeutet, eine Verbindung der Formel VI

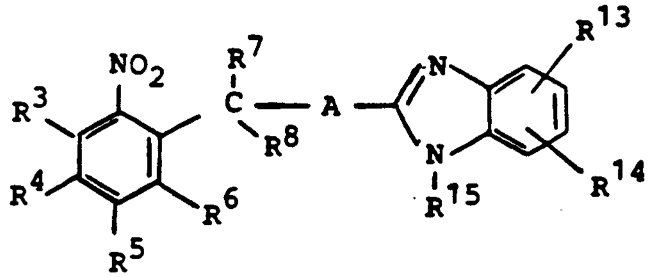

(VI)

in welcher $R^3$, $R^4$, $R^5$, $R^6$, $R^7$, $R^8$, $R^{13}$, $R^{14}$ und $R^{15}$ wie im Anspruch 1 definiert sind und A für ein Schwefelatom steht, reduziert,

in den nach a), b) oder c) erhaltenen Verbindungen der Formel I, in welcher A für -S- oder SO-

und/oder $R^3$ bis $R^6$ und/oder $R^{13}$, $R^{14}$ für -S- oder -SO- haltige Reste stehen, zur entsprechenden -SO- oder $-SO_2$-Gruppe oxidiert, eine Verbindung der Formel I worin $R^1$ und/oder $R^2$ für Wasserstoff steht, alkyliert oder acyliert,
eine Verbindung der Formel I, worin $R^1$ und/oder $R^2$ für Acyl steht, einer desacylierenden Hydrolyse oder Hydrogenolyse unterWirft,
eine so erhaltene Verbindung der Formel I gegebenenfalls in ihr physiologisch verträgliches Salz überführt.

**8.** Verbindung gemäß einem der Ansprüche 1 bis 6 zur Anwendung als Heilmittel.

**9.** Verbindung gemäß einem der Ansprüche 1 bis 6 zur Anwendung als Magensäuresekretionshemmer.

**10.** Pharmazeutisches Mittel enthaltend eine Verbindung gemäß einem der Ansprüche 1 bis 6 .

**Patentansprüche für folgenden Vertragsstaat : AT**

**1.** Verfahren zur Herstellung einer Verbindung der Formel I

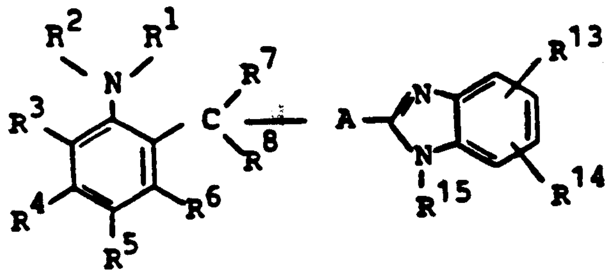

(I)

in welcher

| | |
|---|---|
| A | für -S- oder -SO- steht, |
| $R^1$ | $(C_1-C_4)$-Alkyl, vorzugsweise Methyl oder Ethyl bedeutet, |
| $R^2$, $R^7$, $R^8$ und $R^{15}$ | jeweils Wasserstoff bedeuten, |
| $R^3$,$R^4$,$R^5$ und $R^6$ | gleich oder verschieden sind und Wasserstoff, $(C_1-C_4)$-Alkyl, oder Methoxy bedeuten oder $R^4$ und $R^5$ zusammen für -CH=CH-CH=CH- stehen, |
| $R^{13}$ und $R^{14}$ | gleich oder verschieden sind und $(C_1-C_4)$-Alkoxy bedeuten, |

sowie deren physiologisch verträglichen Salzen, dadurch gekennzeichnet, daß man
a) eine Verbindung der Formel II

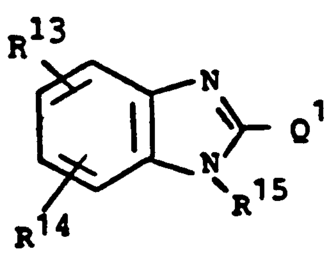

(II)

in welcher $R^{13}$, $R^{14}$ und $R^{15}$ wie in Anspruch 1 definiert sind und
$Q^1$ i. eine Abgangsgruppe oder
ii. -SH, $-S^-$ oder $-SO_2^-$ bedeutet,
umsetzt mit einer Verbindung der Formel III

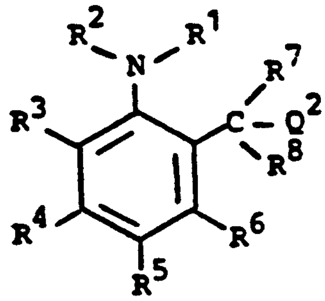

(III)

in welcher $R^1$, $R^2$, $R^3$, $R^4$, $R^5$, $R^6$, $R^7$ und $R^8$ wie im Anspruch 1 definiert sind, und

$Q^2$ im obengenannten Fall i. -SH, $-S^-$ oder $-SO_2^-$,
im obengenannten Fall ii. eine Angangsgruppe bedeutet,

b) eine Verbindung der Formel IV

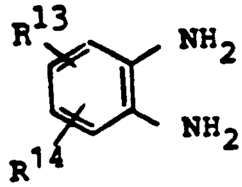

(IV)

in welcher $R^{13}$ und $R^{14}$ wie oben definiert sind, umsetzt, mit einer Verbindung der Formel V

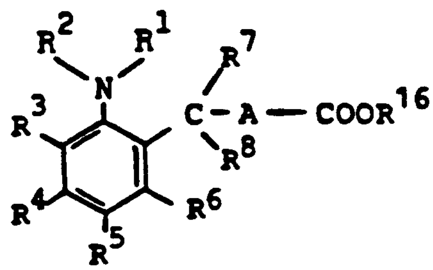

(V)

in welcher $R^1$, $R^2$, $R^3$, $R^4$, $R^5$, $R^6$, $R^7$, $R^8$ und A wie oben definiert sind und
$R^{16}$ für eine veresternde Gruppe steht, oder

c) zur Herstellung einer Verbindung der Formel I, worin $R^1$ und $R^2$ jeweils Wasserstoff bedeutet, eine Verbindung der Formel VI

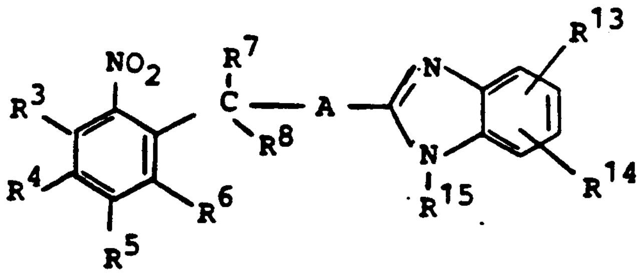

(VI)

in welcher $R^3$, $R^4$, $R^5$, $R^6$, $R^7$, $R^8$, $R^{13}$, $R^{14}$ und $R^{15}$ wie im Anspruch 1 definiert sind und A für ein Schwefelatom steht, reduziert,

in den nach a), b) oder c) erhaltenen Verbindungen der Formel I, in welcher A für -S- oder SO- und/oder $R^3$ bis $R^6$ und/oder $R^{13}$, $R^{14}$ für -S- oder -SO- haltige Reste stehen, zur entsprechenden -SO- oder $-SO_2$-Gruppe oxidiert, eine Verbindung der Formel I worin $R^1$ und/oder $R^2$ für Wasserstoff steht,

alkyliert oder acyliert,
eine Verbindung der Formel I, worin $R^1$ und/oder $R^2$ für Acyl steht, einer desacylierenden Hydrolyse oder Hydrogenolyse unterwirft,
eine so erhaltene Verbindung der Formel I gegebenenfalls in ihr physiologisch verträgliches Salz überführt.

**2.** Verfahren gemäß Anspruch 1 , dadurch gekennzeichnet, daß eine Verbindung der Formel I hergestellt wird, in welcher A für -SO- steht, oder deren physiologisch verträglichen Salze.

**3.** Verfahren gemäß Anspruch 1 oder 2, dadurch gekennzeichnet, daß eine Verbindung der Formel I hergestellt wird, in welcher $R^3$, $R^4$, $R^5$ und $R^6$ jeweils für Wasserstoff stehen, oder deren physiologisch verträglichen Salze.

**4.** Verfahren gemäß einem der Ansprüche 1 bis 3, dadurch gekennzeichnet, daß eine Verbindung der Formel I hergestellt wird, in welcher $R^{13}$ und $R^{14}$ in der 5- bzw. 6-Position des Benzimidazol-Systems stehen, gleich oder verschieden sind und Methoxy oder Ethoxy, vorzugsweise Methoxy bedeuten und $R^1$ Ethyl bedeutet,
oder deren physiologisch verträglichen Salze.

**5.** Verfahren gemäß Anspruch 1 zur Herstellung von 2-(2-Ethylamino-benzylsulfinyl)-5,6-dimethoxybenzimidazol und dessen physiologisch verträglichen Salzen.

**6.** Verfahren gemäß einem der Ansprüche 1 bis 4 , dadurch gekennzeichnet, daß eine Verbindung der Formel I hergestellt wird, in welcher ein oder zwei der Reste $R^3$, $R^4$, $R^5$ und $R^6$ für $(C_1\text{-}C_4)$-Alkyl steht (stehen),
oder deren physiologisch verträglichen Salze.

**Claims**

**Claims for the following Contracting States : BE, CH, DE, FR, GB, IT, LI, LU, NL, SE**

**1.** A compound of the formula I

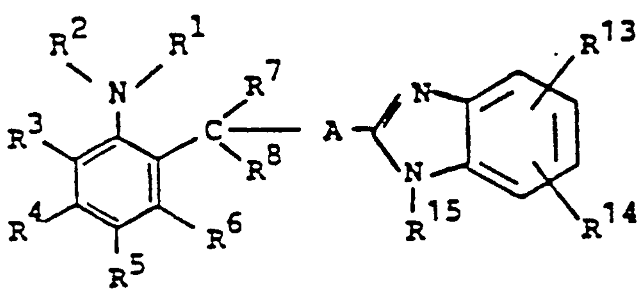

(I)

in which
A represents -S- or -SO-,
$R^1$ denotes $(C_1\text{-}C_4)$-alkyl, preferably methyl or ethyl,
$R^2$, $R^7$, $R^8$ and $R^{15}$ each denote hydrogen,
$R^3$, $R^4$, $R^5$, and $R^6$ are identical or different and denote hydrogen, $(C_1\text{-}C_4)$-alkyl or methoxy, or $R^4$ and $R^5$ together represent -CH=CH-CH=CH-,
$R^{13}$ and $R^{14}$ are identical or different and denote $(C_1\text{-}C_4)$-alkoxy,
and its physiologically tolerated salts

**2.** A compound of the formula I as claimed in claim 1, in which A represents -SO-,
and its physiologically tolerated salts

**3.** A compound of the formula I as claimed in claim 1 or 2, in which $R^3$, $R^4$, $R^5$ and $R^6$ each represent hydrogen, and its physiologically tolerated salts.

**4.** A compound of the formula I as claimed in one of claims 1 to 3, in which $R^{13}$ and $R^{14}$ are located in the 5 position and 6 position of the benzimidazole system, are identical or different, and denote methoxy or ethoxy, preferably methoxy, and $R^1$ denotes ethyl, and its physiologically tolerated salts.

**5.** 2-(2-Ethylaminobenzylsulfinyl)-5,6-dimethoxybenzimidazole and its physiologically tolerated salts.

**6.** A compound of the formula I as claimed in one of claims 1 to 4, in which one or two of the radicals $R^3$, $R^4$, $R^5$ and $R^6$ represents (represent) $(C_1$-$C_4)$-alkyl, and its physiologically tolerated salts.

**7.** A process for the preparation of a compound of the formula I as claimed in one of claims 1 to 6, which comprises

a) reaction of a compound of the formula II

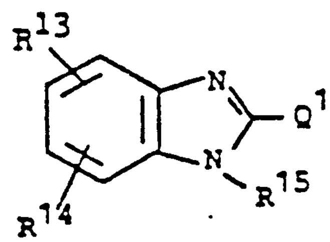

(II)

in which $R^{13}$, $R^{14}$ and $R^{15}$ are as defined in claim 1, and

$Q^1$ denotes i. a leaving group or

ii. -SH, $-S^-$ or $-SO_2^-$,

with a compound of the formula III

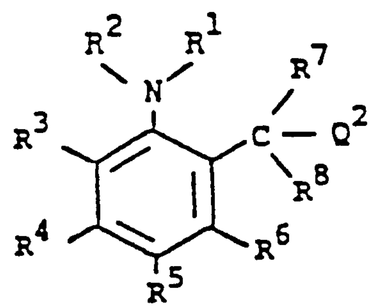

(III)

in which $R^1$, $R^2$, $R^3$, $R^4$, $R^5$, $R^6$, $R^7$ and $R^8$ are as defined in claim 1, and

$Q^2$ denotes, in the abovementioned case i., -SH, $-S^-$ or $-SO_2^-$, and, in the abovementioned case ii., a leaving group,

b) reaction of a compound of the formula IV

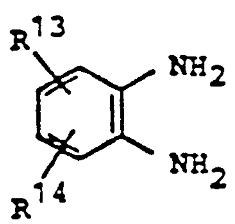

(IV)

in which $R^{13}$ and $R^{14}$ are as defined above, with a compound of the formula V

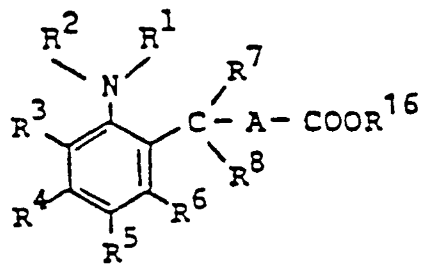

(V)

in which $R^1$, $R^2$, $R^3$, $R^4$, $R^5$, $R^6$, $R^7$, $R^8$ and A are as defined above, and $R^{16}$ represents an esterifying group, or

c) for the preparation of a compound of the formula I in which $R^1$ and $R^2$ each denote hydrogen, reduction of a compound of the formula VI

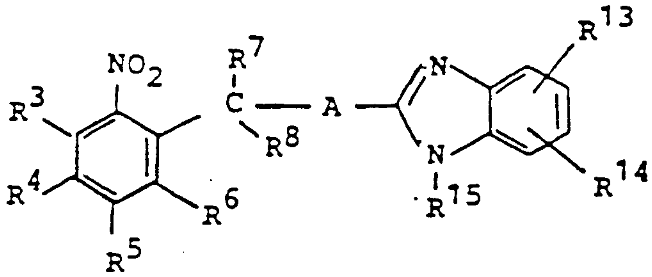

(VI)

in which $R^3$, $R^4$, $R^5$, $R^6$, $R^7$, $R^8$, $R^{13}$, $R^{14}$, and $R^{15}$ are as defined in claim 1, and A represents a sulfur atom, oxidation, in the compounds of the formula I which have been obtained according to a), b) or c) and in which A represents -S- or -SO-, and/or $R^3$ to $R^6$ and/or $R^{13}$ and $R^{14}$ represent radicals containing -S- or -SO-, to the corresponding -SO- or $-SO_2-$ group, alkylation or acylation of a compound of the formula I in which $R^1$ and/or $R^2$ represents hydrogen, subjection of a compound of the formula I in which $R^1$ and/or $R^2$ represents acyl to deacylating hydrolysis or hydrogenolysis, and conversion of a compound of the formula I, thus obtained, where appropriate into its physiologically tolerated salt.

**8.** A compound as claimed in one of claims 1 to 6, for use as medicine.

**9.** A compound as claimed in one of claims 1 to 6, for use as inhibitor of gastric acid secretion.

**10.** A pharmaceutical agent containing a compound as claimed in one of claims 1 to 6.

**Claims for the following Contracting State : AT**

**1.** A process for the preparation of a compound of the formula I

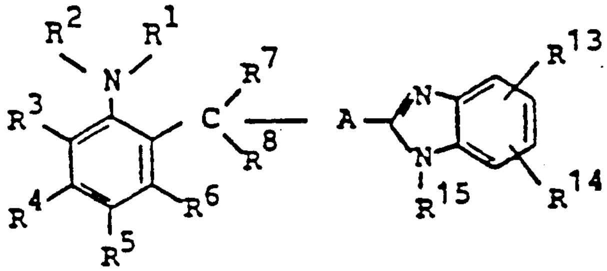

(I)

in which

A represents -S- or -SO-,
$R^1$ denotes $(C_1\text{-}C_4)$-alkyl, preferably methyl or ethyl, $R^2$, $R^7$, $R^8$ and $R^{15}$ each denote hydrogen,
$R^3$, $R^4$, $R^5$ and $R^6$ are identical or different and denote hydrogen, $(C_1\text{-}C_4)$-alkyl or methoxy, or $R^4$ and $R^5$ together represent -CH = CH-CH = CH-,
$R^{13}$ and $R^{14}$ are identical or different and denote $(C_1\text{-}C_4)$ alkoxy,
and of its physiologically tolerated salts, which comprises

a) reaction of a compound of the formula II

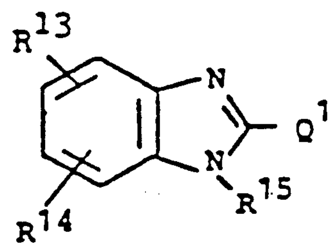

(II)

in which $R^{13}$, $R^{14}$ and $R^{15}$ are as defined in claim 1, and
$Q^1$ denotes i. a leaving group or
ii. -SH, $-S^-$ or $-SO_2$-,
with a compound of the formula III

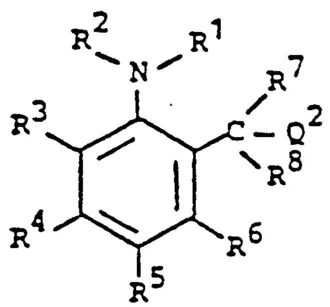

(III)

in which $R^1$, $R^2$, $R^3$, $R^4$, $R^5$, $R^6$, $R^7$, and $R^8$ are as defined in claim 1, and
$Q^2$ denotes, in the abovementioned case i., -SH, $-S^-$ or $-SO_2^-$, and, in the abovementioned case ii., a leaving group,

b) reaction of a compound of the formula IV

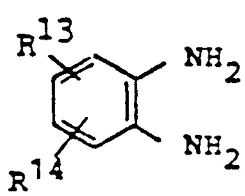

(IV)

in which $R^{13}$ and $R^{14}$ are as defined above, with a compound of the formula V

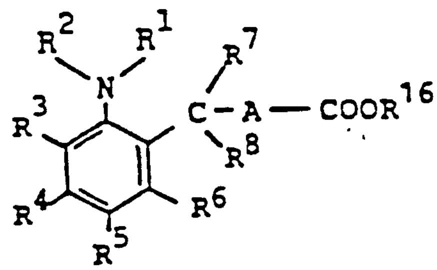

(V)

in which $R^1$, $R^2$, $R^3$, $R^4$, $R^5$, $R^6$, $R^7$, $R^8$ and A are as defined above, and $R^{16}$ represents an esterifying group, or
c) for the preparation of a compound of the formula I in which $R^1$ and $R^2$ each denote hydrogen, reduction of a compound of the formula VI

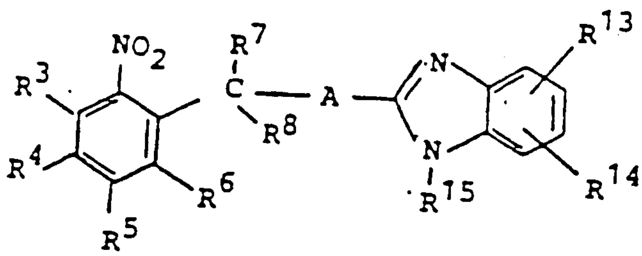

(VI)

in which $R^3$, $R^4$, $R^5$, $R^6$, $R^7$, $R^8$, $R^{13}$, $R^{14}$ and $R^{15}$ are as defined in claim 1, and A represents a sulfur atom, oxidation, in the compounds of the formula I which have been obtained according to a), b) or c) and in which A represents -S- or -SO-, and/or $R^3$ to $R^6$ and/or $R^{13}$ and $R^{14}$ represent radicals containing -S-or -SO-, to the corresponding -SO- or $-SO_2-$ group, alkylation or acylation of a compound of the formula I in which $R^1$ and/or $R^2$ represents hydrogen, subjection of a compound of the formula I in which $R^1$ and/or $R^2$ represents acyl to deacylating hydrolysis or hydrogenolysis, and conversion of a compound of the formula I, thus obtained, where appropriate into its physiologically tolerated salt.

2. The process as claimed in claim 1, wherein there is prepared a compound of the formula I in which A represents -SO-, or its physiologically tolerated salts.

3. The process as claimed in claim 1 or 2, wherein there is prepared a compound of the formula I in which $R^3$, $R^4$, $R^5$ and $R^6$ each represent hydrogen, or its physiologically tolerated salts.

4. The process as claimed in one of claims 1 to 3, wherein there is prepared a compound of the formula I in which $R^{13}$ and $R^{14}$ are located in the 5 position and 6 position of the benzimidazole system, are identical or different, and denote methoxy or ethoxy, preferably methoxy, and $R^1$ denotes ethyl, or its physiologically tolerated salts.

5. The process as claimed in claim 1 for the preparation of 2-(2-ethylaminobenzylsulfinyl)-5,6-dimethoxybenzimidazole and its physiologically tolerated salts.

6. The process as claimed in one of claims 1 to 4, wherein there is prepared a compound of the formula I in which one or two of the radicals $R^3$, $R^4$, $R^5$ and $R^6$ represents (represent) $(C_1$-$C_4)$-alkyl, or its physiologically tolerated salts.

**Revendications**

**Revendications pour les Etats contractants suivants : BE, CH, DE, FR, GB, IT, LI, LU, NL, SE**

1. Composé de formule I

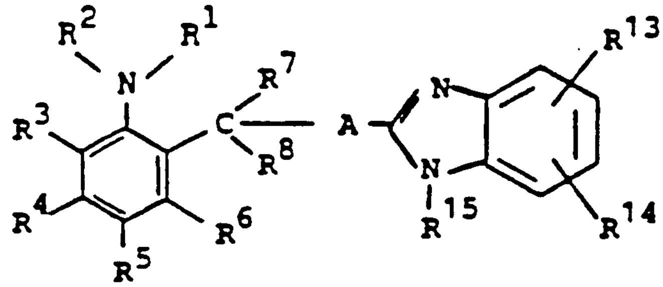

(I)

dans laquelle

| | |
|---|---|
| A | représente -S- ou -SO-, |
| $R^1$ | représente un groupe alkyle en $C_1$-$C_4$, de préférence le groupe méthyle ou éthyle, |
| $R^2$, $R^7$, $R^8$ et $R^{15}$ | représentent chacun un atome d'hydrogène, |
| $R^3$, $R^4$, $R^5$ et $R^6$ | sont identiques ou différents, et représentent un atome atome d'hydrogène ou un groupe alkyle en $C_1$-$C_4$ ou méthoxy, ou $R^4$ et $R^5$ représentent ensemble -CH=CH-CH=CH-, |
| $R^{13}$ et $R^{14}$ | sont identiques ou différents, et représentent un groupe alcoxy en $C_1$-$C_4$. |

et sels physiologiquement acceptables de celui-ci.

**2.** Composé de formule I selon la revendication 1, dans lequel A représente -SO-, et sels physiologiquement acceptables de celui-ci.

**3.** Composé de formule I selon la revendication 1 ou 2, dans lequel $R^{3,}$ $R^4$, $R^5$ et $R^6$ représentent chacun un atome d'hydrogène, et sels physiologiquement acceptable de celui-ci.

**4.** Composé de formule I selon l'une des revendications 1 à 3, dans lequel $R^{13}$ et $R^{14}$ sont en position 5 ou 6, respectivement, du système benzimidazole, sont identiques ou différents et représentent le groupe méthoxy ou éthoxy, de préférence le groupe m éthoxy, et $R^1$ représente le groupe éthyle, et sels physiologiquement acceptables de celui-ci.

**5.** 2-(2-éthylamino-benzylsulfinyl)-5,6-diméthoxybenzimidazole et sels physiologiquement acceptables de celui-ci.

**6.** Composé de formule I selon l'une des revendications 1 à 4, dans lequel un ou deux des radicaux $R^3$, $R^4$, $R^5$ et $R^6$ représente(nt) un groupe alkyle en $C_1$-$C_4$, et sels physiologiquement acceptable de celui-ci.

**7.** Procédé pour la préparation d'un composé de formule I selon l'une des revendications 1 à 6, caractérisé en ce que

a) on fait réagir un composé de formule II

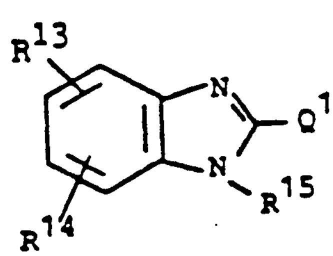

(II)

dans laquelle $R^{13}$, $R^{14}$ et $R^{15}$ sont tels que définis dans la revendication 1, et

$Q^1$ représente I. un groupe séparable ou
II. -SH, $-S^-$ ou $-SO_2^-$,

avec un composé de formule III

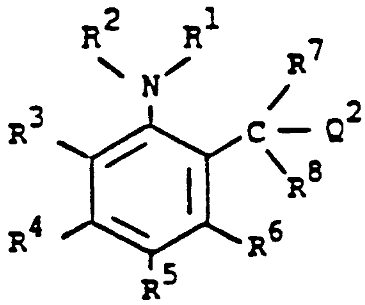

(III)

dans laquelle $R^1$, $R^2$, $R^3$, $R^4$, $R^5$, $R^6$, $R^7$ et $R^8$ sont tels que définis dans la revendication 1, et

$Q^2$ représente

dans le cas I mentionné ci-dessus, -SH, $-S^-$ ou $-SO_2^-$,

dans le cas II mentionné ci-dessus, un groupe séparable,

b) on fait réagir un composé de formule IV

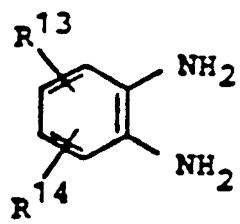

(IV)

dans laquelle $R^{13}$ et $R^{14}$ sont tels que définis plus haut, avec un composé de formule V

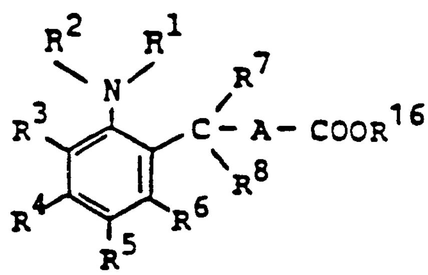

(V)

dans laquelle $R^1$, $R^2$, $R^3$, $R^4$, $R^5$, $R^6$, $R^7$, $R^8$ et A sont tels que définis plus haut, et $R^{16}$ représente un groupe estérifiant, ou

c) pour la préparation d'un composé de formule I dans lequel $R^1$ et $R^2$ représentent chacun un atome d'hydrogène, on réduit un composé de formule VI

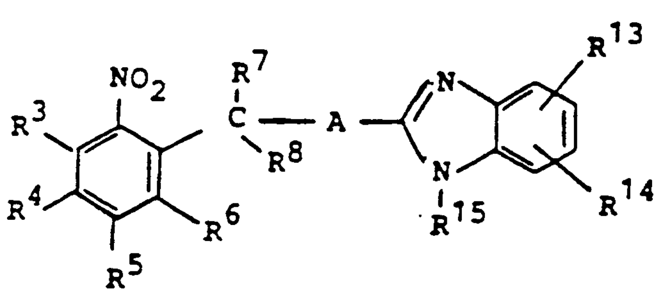

(VI)

dans laquelle $R^3$, $R^4$, $R^5$, $R^6$, $R^7$, $R^8$, $R^{13}$, $R^{14}$ et $R^{15}$ sont tels que définis dans la revendication 1, et A représente un atome de soufre,

dans les composés de formule I obtenus selon a), b) ou c), dans lesquels A représente -S- ou -SO- et/ou $R^3$ à $R^6$ et/ou $R^{13}$, $R^{14}$ représentent des radicaux contenant -S- ou -SO-, on les oxyde en le groupe -SO- ou $-SO_2-$ correspondant,
on soumet à une alkylation ou à une acylation un composé de formule I dans lequel $R^1$ et/ou $R^2$ représente(nt) un atome d'hydrogène,
on soumet à une hydrogénolyse ou hydrolyse désacylante un composé de formule I dans lequel $R^1$ et/ou $R^2$ représente(nt) un groupe acyle,
éventuellement on convertit un composé de formule I ainsi obtenu en un de ses sels physiologiquement acceptables.

**8.** Composé selon l'une des revendications 1 à 6, destiné à être utilisé en tant que médicament.

**9.** Composé selon l'une des revendications 1 à 6,destiné à être utilisé en tant qu'inhibiteur de la sécrétion d'acide gastrique.

**10.** Produit pharmaceutique contenant un composé selon l'une des revendications 1 à 6.

**Revendications pour l'Etat contractant suivant : AT**

**1.** Procédé pour la préparation d'un composé de formule I

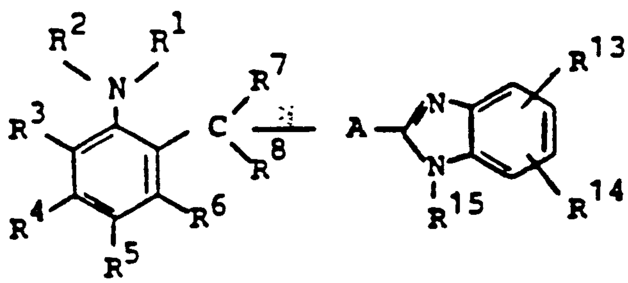

(I)

dans laquelle

| | |
|---|---|
| A | représente -S- ou -SO-, |
| $R^1$ | représente un groupe alkyle en $C_1$-$C_4$, de préférence le groupe méthyle ou éthyle, |
| $R^2$, $R^7$, $R^8$ et $R^{15}$ | représentent chacun un atome d'hydrogène, |
| $R^3$, $R^4$, $R^5$ et $R^6$ | sont identiques ou différents, et représentent un atome atome d'hydrogène ou un groupe alkyle en $C_1$-$C_4$ ou méthoxy, ou $R^4$ et $R^5$ représentent ensemble -CH=CH-CH=CH-, |
| $R^{13}$ et $R^{14}$ | sont identiques ou différents, et représentent un groupe alcoxy en $C_1$-$C_4$. |

ainsi que de ses sels physiologiquement acceptables, caractérisé en ce que
a) on fait réagir un composé de formule II

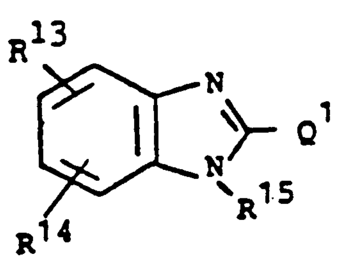

(II)

dans laquelle $R^{13}$, $R^{14}$ et $R^{15}$ sont tels que définis plus haut, et
$Q^1$ représente I. un groupe séparable ou
II. -SH, $-S^-$ ou $-SO_2^-$,
avec un composé de formule III

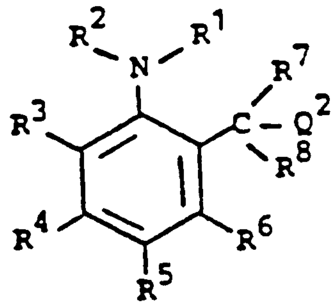

(III)

dans laquelle $R^1$, $R^2$, $R^3$, $R^4$, $R^5$, $R^6$, $R^7$ et $R^8$ sont tels que définis plus haut, et

$Q^2$ représente

dans le cas I mentionné ci-dessus, -SH, $-S^-$ ou $-SO_2^-$,

dans le cas II mentionné ci-dessus, un groupe séparable,

b) on fait réagir un composé de formule IV

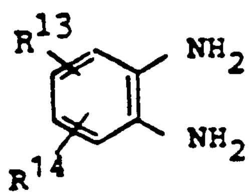

(IV)

dans laquelle $R^{13}$ et $R^{14}$ sont tels que définis plus haut, avec un composé de formule V

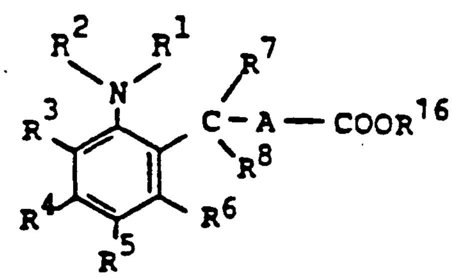

(V)

dans laquelle $R^1$, $R^2$, $R^3$, $R^4$, $R^5$, $R^6$, $R^7$, $R^8$ et A sont tels que définis plus haut, et $R^{16}$ représente un groupe estérifiant, ou

c) pour la préparation d'un composé de formule I dans lequel $R^1$ et $R^2$ représentent chacun un atome d'hydrogène, on réduit un composé de formule VI

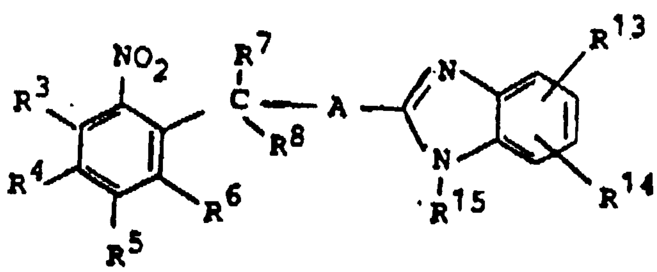

(VI)

dans laquelle $R^3$, $R^4$, $R^5$, $R^6$, $R^7$, $R^8$, $R^{13}$, $R^{14}$ et $R^{15}$ sont tels que définis plus haut, et A représente un atome de soufre,

dans les composés de formule I obtenus selon a), b) ou c), dans lesquels A représente -S- ou -SO- et/ou $R^3$ à $R^6$ et/ou $R^{13}$, $R^{14}$ représentent des radicaux contenant -S- ou -SO-, on les oxyde en le

groupe -SO- ou $-SO_2-$ correspondant,
on soumet à une alkylation ou à une acylation un composé de formule I dans lequel $R^1$ et/ou $R^2$ représente(nt) un atome d'hydrogène,
on soumet à une hydrogénolyse ou hydrolyse désacylante un composé de formule I dans lequel $R^1$ et/ou $R^2$ représente(nt) un groupe acyle,
éventuellement on convertit un composé de formule I ainsi obtenu en un de ses sels physiologiquement acceptables.

**2.** Procédé selon la revendication 1, caractérisé en ce que l'on prépare un composé de formule I dans lequel A représente -SO-, ou ses sels physiologiquement acceptables.

**3.** Procédé selon la revendication 1 ou 2, caractérisé en ce que l'on prépare un composé de formule I dans lequel $R^3$, $R^4$, $R^5$ et $R^6$ représentent chacun un atome d'hydrogène, ou ses sels physiologiquement acceptables.

**4.** Procédé selon l'une des revendications 1 à 3, caractérisé en ce que l'on prépare un composé de formule I dans lequel $R^{13}$ et $R^{14}$ sont en position 5 ou 6, respectivement, du système benzimidazole, sont identiques ou différents et représentent le groupe méthoxy ou éthoxy, de préférence le groupe m éthoxy, et $R^1$ représente le groupe éthyle, ou ses sels physiologiquement acceptables.

**5.** Procédé selon la revendication 1, pour la préparation du 2-(2-éthylamino-benzylsulfinyl)-5,6-diméthoxybenzimidazole et de ses sels physiologiquement acceptables.

**6.** Procédé selon l'une des revendications 1 à 4, caractérisé en ce que l'on prépare un composé de formule I dans lequel un ou deux des radicaux $R^3$, $R^4$, $R^5$ et $R^6$ représente(nt) un groupe alkyle en $C_1$-$C_4$, ou ses sels physiologiquement acceptables.